Europäisches Patentamt

European Patent Office

Office européen des brevets

(19)

(11) **EP 0 808 300 B1**

(12) **FASCICULE DE BREVET EUROPEEN**

(45) Date de publication et mention
de la délivrance du brevet:
**16.08.2000 Bulletin 2000/33**

(51) Int Cl.[7]: **C07C 17/38**, C07C 17/386,
C07C 19/08, C07C 19/12

(21) Numéro de dépôt: **96903059.2**

(22) Date de dépôt: **06.02.1996**

(86) Numéro de dépôt international:
**PCT/FR96/00196**

(87) Numéro de publication internationale:
**WO 96/24569 (15.08.1996 Gazette 1996/37)**

(54) **PROCEDE DE PURIFICATION DU PENTAFLUOROETHANE**

VERFAHREN ZUR REINIGUNG VON PENTAFLUORETHAN

METHOD FOR PURIFYING PENTAFLUOROETHANE

(84) Etats contractants désignés:
**AT BE CH DE DK ES FR GB GR IE IT LI LU NL PT
SE**

(30) Priorité: **07.02.1995 FR 9501381**

(43) Date de publication de la demande:
**26.11.1997 Bulletin 1997/48**

(73) Titulaire: **ELF ATOCHEM S.A.**
**92800 Puteaux, Hauts-de-Seine (FR)**

(72) Inventeurs:
• **GUIRAUD, Emmanuel**
**F-69230 Saint-Genis-Laval (FR)**

• **DESCAMPS, Cathy**
**F-69450 Saint-Cyr-au-Mont-d'Or (FR)**

(74) Mandataire: **Leboulenger, Jean**
**Elf Atochem S.A.,**
**Département Propriété Industrielle,**
**Cedex 42 - La Défense 10**
**92091 Paris la Défense (FR)**

(56) Documents cités:
**EP-A- 0 472 391        WO-A-95/21147**

• **RESEARCH DISCLOSURE, vol. 360, 1994
HAVANT GB, pages 191-193, 'Methods for
Separating Chloro-Carbons from
Hydrofluoroalkanes'**

**Description**

L'invention concerne la purification du pentafluoroéthane (F125) contenant du chloropentafluoroéthane (F115) et a plus particulièrement pour objet un procédé de purification dans lequel le F115 est éliminé par extraction liquide-liquide ou par distillation extractive et facilement récupéré en vue de sa transformation ultérieure en produits inoffensifs pour l'atmosphère terrestre.

Le pentafluoroéthane est l'un des substituts possibles des chlorofluorocarbures (CFC) qui sont concernés par le protocole de Montréal et se caractérisent par une durée de vie exceptionnellement élevée leur permettant d'atteindre les hautes couches de l'atmosphère et de participer ainsi sous l'influence du rayonnement UV à la destruction de la couche d'ozone. Il est donc évident que leurs substituts ne devront, en fonction des divers procédés d'obtention, ne renfermer que des traces de ces CFC.

Les substituts sont généralement obtenus soit par des méthodes de fluoruration appropriées qui ne sont pas hautement sélectives et peuvent générer par dismutation des composés perhalogénés du type CFC, soit à partir de CFC eux-mêmes par des méthodes de réduction, en pratique par des réactions d'hydrogénolyse. C'est ainsi que le pentafluoroéthane (F125) peut être préparé par fluoration du perchloroéthylène ou de ses produits de fluoration intermédiaires tels que le dichlorotrifluoroéthane (F123) et le chlorotétrafluoroéthane (F124), ou par hydrogénolyse du chloropentafluoroéthane (F115). Dans les deux cas , le F125 produit contient des quantités non négligeables de F115 qu'il convient, le F115 étant un CFC, d'éliminer le plus complètement possible.

Or, l'existence d'un azéotrope F115/F125 à 21 % en poids de F115 (voir le brevet US 3 505 233) avec un point d'ébullition (-48,5°C sous 1,013 bar) très voisin de celui du F125 (-48,1°C) rend pratiquement impossible la séparation complète du F115 et du F125 par distillation. L'élimination du F115 dans le F125 ne peut donc se faire que par voie chimique ou par des méthodes physiques mettant en jeu un tiers corps.

Dans la demande de brevet EP 508 631 qui décrit la production d'hydrofluorocarbures (HFC) par réduction chimique en phase liquide de composés chlorés, bromés ou iodés avec un hydrure métallique ou un complexe d'un tel hydrure, il est indiqué que ce procédé peut être intéressant pour purifier certains HFC comme le F125. Dans le même but, la demande de brevet japonais publiée (Kokai) sous le n°2001414/90 utilise des couples rédox métalliques en milieu solvant. D'autres techniques comme celle décrite dans Journal of Fluorine Chemistry, 1991 vol. 55, p.105-107, utilisent des réducteurs organiques tels que le formiate d'ammonium en milieu DMF et en présence de persulfate d'ammonium.

Ces procédés qui font appel à des réactifs difficiles à manipuler (hydrures métalliques) ou susceptibles de poser des problèmes d'effluents, sont peu compatibles avec une production industrielle de F125 en tonnages importants.

Pour une fabrication industrielle de F125, la technique de distillation extractive apparaît être un procédé idéal pour éliminer le F115 résiduel.

Dans un procédé de distillation extractive, la séparation des constituants d'un mélange binaire se fait à l'aide d'une colonne dite d'extraction comportant successivement, du bouilleur à la tête, trois tronçons, l'un d'épuisement, le second d'absorption et le troisième de récupération.

Le mélange binaire à fractionner est injecté en tête du tronçon d'épuisement alors que le tiers corps jouant le rôle de solvant sélectif est introduit en tête du tronçon d'absorption de manière à circuler à l'état liquide de son point d'introduction jusqu'au bouilleur.

Le troisième tronçon dit de récupération sert à séparer par distillation le constituant le moins absorbé, des traces de solvant entraînées sous l'effet de sa tension de vapeur non nulle.

L'application de cette technique à la purification du 1,1,1,2-tétrafluoroéthane (F134a) a fait l'objet du brevet US 5 200 431 ; l'agent d'extraction utilisé est un solvant chloré ou un hydrocarbure aliphatique.

L'application de la distillation extractive à la purification du F125 est déjà décrite dans le brevet US 5 087 329 qui utilise comme agent d'extraction un hydrocarbure fluoré en $C_1$ à $C_4$ contenant éventuellement des atomes d'hydrogène et/ou de chlore et ayant un point d'ébullition compris entre -39 et +50°C. D'après les données de ce brevet, les dichlorotétrafluoroéthanes (F114 et F114a) sont au moins trois fois plus efficaces que les autres composés cités. D'autre part, 5 des 8 solvants cités sont des CFC concernés par le protocole de Montréal et dont la commercialisation devrait cesser dans un proche avenir.

L'utilisation industrielle du procédé selon ce brevet ne peut donc être économiquement envisagée que lorsque l'agent d'extraction mis en oeuvre fait partie de la chaîne d'intermédiaires conduisant au F125, c'est-à-dire en fait dans les procédés de préparation du F125 par hydrogénolyse.

Dans le cas de fabrications du F125 par fluoruration du perchloroéthylène ou de ses produits de fluoration partielle (F122, F123, F124), le brevet US 5 087 329 ne laisse le choix qu'entre des CFC que l'on ne trouvera plus sur le marché et des produits moins performants tels que le F124 ou le F123.

Il a maintenant été trouvé que le perchloréthylène présente une sélectivité très supérieure à celle des chlorofluoroéthanes et qu'en outre il existe un large domaine de décantation des mélanges F125/F115/perchloréthylène.

La décantation de ces mélanges permet d'obtenir :

- une phase inférieure riche en perchloréthylène contenant du F125 enrichi en F115 par rapport au mélange de F125 + F115 de départ à traiter,
- une phase supérieure riche en F125 et appauvri en F115 par rapport au mélange F125 + F115 de départ à traiter.

Le ratio F125/F115 s'établit donc dans les deux phases de la manière suivante :

Phase supérieure > mélange initial > phase inférieure

La présente invention a donc pour objet un procédé de purification d'un pentafluoroéthane contenant du chloro-pentafluoroéthane par distillation extractive ou par extraction liquide-liquide, caractérisé en ce que l'on utilise le perchloréthylène comme agent d'extraction.

L'utilisation de perchloréthylène comme solvant d'extraction selon l'invention est particulièrement intéressante à appliquer lorsque l'on veut purifier un F125 obtenu par fluoruration. Dans ce cas en effet, le perchloréthylène utilisé comme solvant d'extraction n'est autre que la matière première utilisée dans le procédé d'obtention du F125.

Le procédé selon l'invention peut être mis en oeuvre selon les principes bien connus de la distillation extractive ou de l'extraction liquide-liquide, en opérant sous des pressions comprises entre 2 et 20 bars absolus, les températures étant données par les diagrammes d'équilibre liquide-vapeur des constituants individuels et de leurs mélanges.

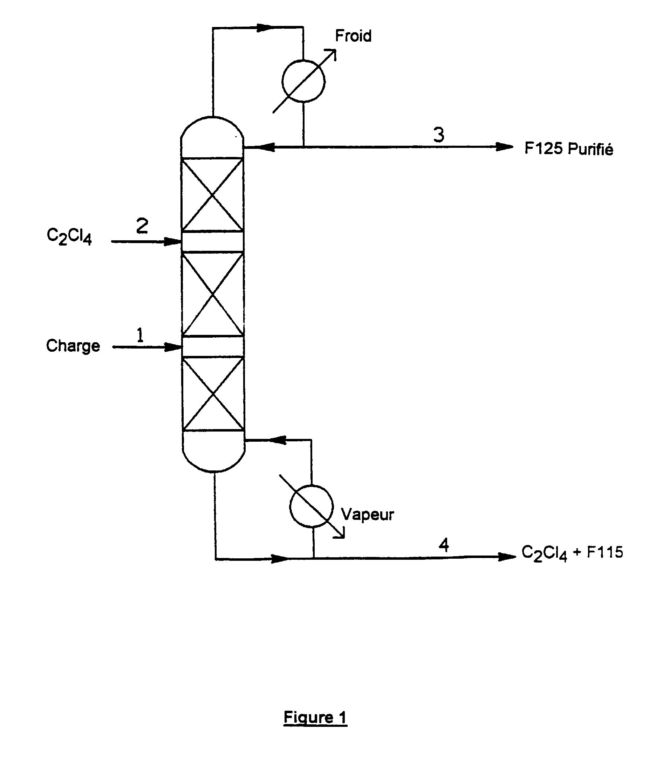

Lorsqu'on opère selon le schéma de la Figure 1 dans une colonne de distillation extractive, la charge (mélange F125-F115 à séparer) est injectée par la conduite (1) en un point situé en tête du tronçon d'épuisement et le perchloréthylène, introduit dans la colonne par la conduite (2) en un point situé en tête du tronçon d'absorption, circule à l'état liquide de son point d'introduction jusqu'au bouilleur. On sort en tête de la colonne de distillation extractive le F125 purifié par la conduite (3) et en pied par la conduite (4) le perchloréthylène enrichi en F115.

Le diamètre et le nombre d'étages de la colonne de distillation extractive, le taux de reflux et les températures et pressions optimales peuvent être facilement calculées par l'homme du métier à partir des données propres aux constituants individuels et à leurs mélanges (volatilités relatives, pressions de vapeur et constantes physiques).

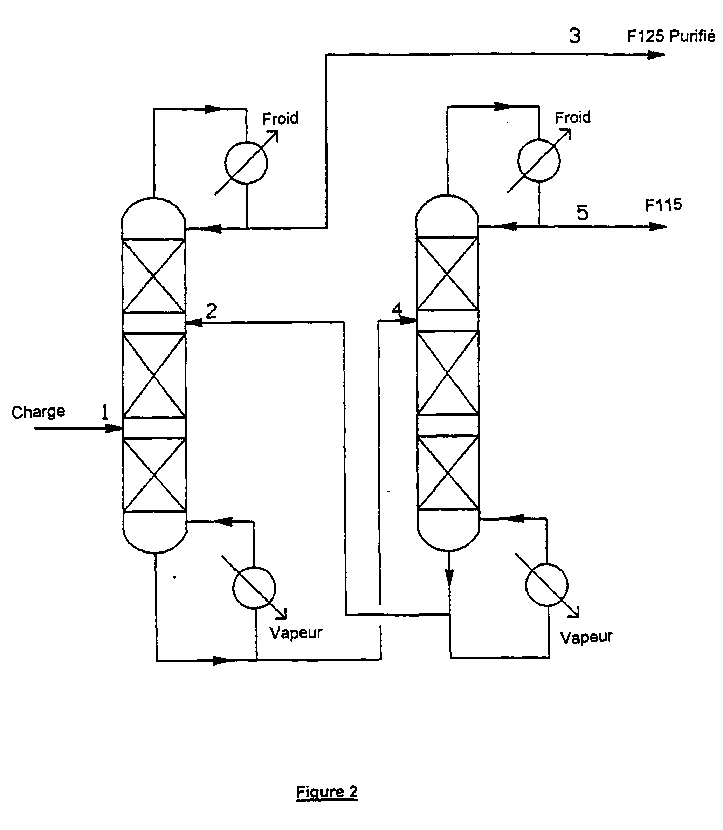

Si l'on souhaite recycler le perchloréthylène, on peut utiliser le dispositif représenté sur la Figure 2 qui combine la colonne de distillation extractive avec une colonne de distillation simple. Le perchloréthylène enrichi en F115 sortant en pied de la colonne de distillation extractive est envoyé par la conduite (4) dans la colonne de distillation simple ; le F115 est récupéré en tête par la conduite (5) et le perchloréthylène est recyclé à la colonne de distillation extractive par la conduite (2).

La séparation du F115 et du F125 par extraction liquide-liquide au moyen de perchloréthylène peut être réalisée selon l'une quelconques des techniques classiques d'extraction liquide-liquide connues de l'homme de l'art (colonne d'extraction, mélangeur-décanteur en série,...).

Les exemples suivants illustrent l'invention sans la limiter.

## EXEMPLE 1

L'aptitude d'un solvant à être utilisé dans la séparation par distillation extractive d'un mélange F115-F125 est appréciée au travers de sa sélectivité (S) définie comme le rapport des solubilités du F115 ($s_{115}$) et du F125 ($s_{125}$) dans le solvant pour une même pression partielle et une même température, soit

$$S = \frac{s_{115}}{s_{125}}$$

Pour déterminer ces solubilités, on utilise un autoclave en acier inox d'une capacité de 477,5 ml (pour les essais comparatifs avec le F114) ou un ballon en verre d'une capacité de 1052 ml (pour les essais avec le perchloréthylène). Après avoir fait le vide dans l'autoclave ou le ballon, on y introduit une pression partielle de F125, de F115 ou d'un mélange gazeux de F125 + F115 de composition connue. On introduit ensuite par piégeage ou coulée directe une quantité connue de solvant et on place l'autoclave (ou le ballon) dans une enceinte thermostatée à 25°C. Après équilibrage, on note la pression totale et on prélève par un dispositif approprié un échantillon de la phase liquide et un échantillon de la phase gazeuse qui sont ensuite analysés par chromatographie en phase gazeuse.

La composition molaire de la phase gazeuse permet de calculer la pression partielle du F115 et/ou du F125. La solubilité du F115 ($s_{115}$) et du F125 ($s_{125}$) dans le solvant, exprimée en g de F115 ou de F125 par litre de solvant en phase liquide, est calculée à partir de la composition molaire de la phase liquide.

En utilisant comme solvant le F114 (à titre comparatif) ou le perchloréthylène (selon l'invention), on a ainsi réalisé les six essais résumés ci-dessous.

***Essai A***

- 333,3 mbar de F125
- 135,8 g de F114

Pression totale = 2224 mbar à 25°C

| | PHASE GAZEUSE | | PHASE LIQUIDE |
|---|---|---|---|
| | *% moles* | *Pression partielle (mbar)* | *% moles* |
| F125 | 4,6 | 102 | 0,49 |
| F114 | 95,4 | 2122 | 99,51 |

Solubilité $s_{125}$ = 5,0 g/l

***Essai B***

- 1000 mbar de F115
- 117,6 g de F114

Pression totale = 2295 mbar à 25°C

| | PHASE GAZEUSE | | PHASE LIQUIDE |
|---|---|---|---|
| | *% moles* | *Pression partielle (mbar)* | *% moles* |
| F115 | 9,46 | 217 | 2,15 |
| F114 | 90,54 | 2078 | 97,85 |

Solubilité $s_{115}$ = 28,9 g/l

***Essai C***

- 1000 mbar de mélange gazeux de F125 + F115 à 3,22 % moles de F115
- 140,4 g de F114

Pression totale = 2394 mbar à 25°C

4

| | PHASE GAZEUSE | | PHASE LIQUIDE |
|---|---|---|---|
| | *% moles* | *Pression partielle (mbar)* | *% moles* |
| F115 | 0,33 | 8 | 0,08 |
| F125 | 12,25 | 293 | 1,55 |
| F114 | 87,42 | 2093 | 98,37 |

Solubilité $s_{115}$ = 1,1 g/l
Solubilité $s_{125}$ = 16,1 g/l

**Essai D**

- 1000 mbar de mélange gazeux de F125 + F115 à 10,0 % moles de F115
- 141,3 g de F114

Pression totale = 2393 mbar à 25°C

| | PHASE GAZEUSE | | PHASE LIQUIDE |
|---|---|---|---|
| | *% moles* | *Pression partielle (mbar)* | *% moles* |
| F115 | 0,91 | 22 | 0,21 |
| F125 | 11,09 | 265 | 1,42 |
| F114 | 88,00 | 2106 | 98,36 |

Solubilité $s_{115}$ = 2,8 g/l
Solubilité $s_{125}$ = 14,8 g/l

**Essai E**

- 889 mbar de mélange gazeux de F125 + F115 à 9,86 % moles de F115
- 162 g de perchloréthylène

Pression totale = 850 mbar à 25°C

|  | PHASE GAZEUSE | | PHASE LIQUIDE |
|  | % moles | Pression partielle (mbar) | % moles |
|---|---|---|---|
| F115 | 8,58 | 73 | 0,15 |
| F125 | 89,82 | 763 | 0,46 |
| Perchloréthylène | 1,6 | 14 | 99,39 |

Solubilité $s_{115}$ = 2,2g/l
Solubilité $s_{125}$ = 5,4 g/l

**_Essai F_**

- 950 mbar de mélange gazeux de F125 + F115 à 3,2 % moles de F115
- 196 g de perchloréthylène

Pression totale = 923 mbar à 25°C

|  | PHASE GAZEUSE | | PHASE LIQUIDE |
|  | % moles | Pression partielle (mbar) | % moles |
|---|---|---|---|
| F115 | 2,68 | 25 | 0,047 |
| F125 | 94,21 | 870 | 0,54 |
| Perchloréthylène | 3,11 | 29 | 99,41 |

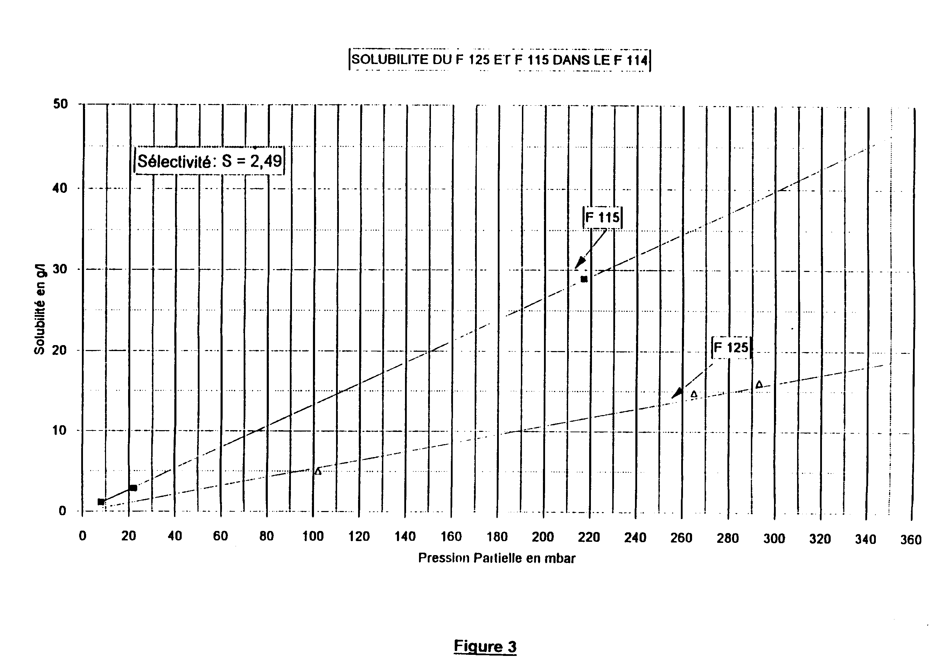

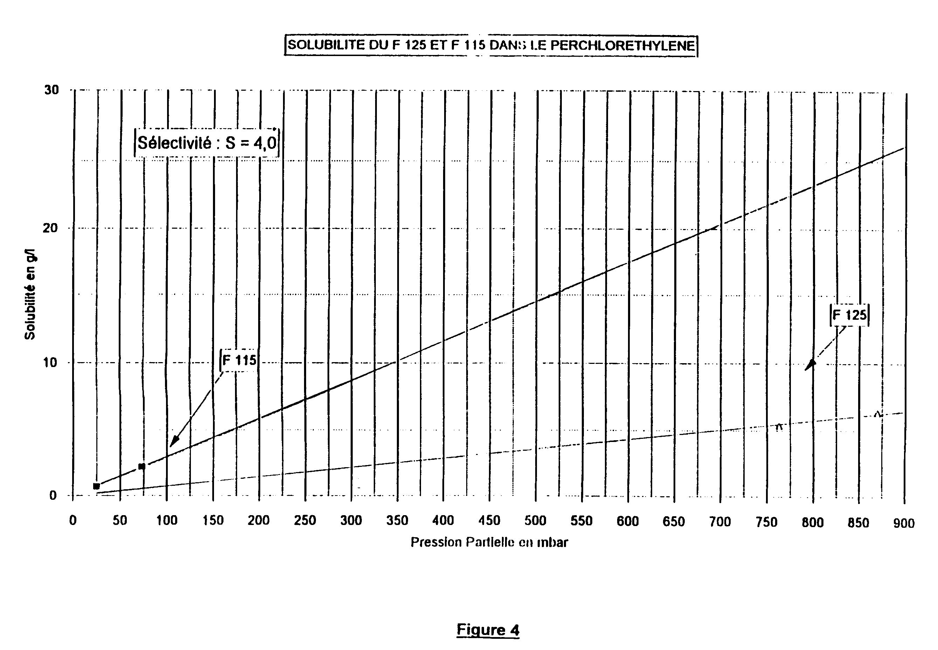

Solubilité $s_{115}$ = 0,7 g/l
Solubilité $s_{125}$ = 6,4 g/l
Ces essais ont permis de tracer les droites de solubilité du F125 et du F115 dans le F114 (Figure 3) et le perchloréthylène (Figure 4) en fonction de la pression partielle en F125 et F115.
La sélectivité obtenue avec le F114 est de 2,49 et est très inférieure à celle (4,0) obtenue avec le perchloréthylène.

## EXEMPLE 2

Pour déterminer le diagramme de phase du ternaire F125/F115/perchloréthylène, on utilise un autoclave en acier Inox de 204 ml équipé de deux tubes plongeurs permettant de prélever un échantillon des deux phases présentes.
Après avoir fait le vide dans l'autoclave, on introduit une quantité connue de perchloréthylène puis de mélange F125/F115 de composition connue, par piégeage ou coulée directe. L'autoclave est ensuite placé dans une enceinte thermostatée à la température désirée et, après équilibrage, on note la pression totale et on analyse par chromatographie en phase gazeuse, la composition des deux phases liquides décantées présentes. On a ainsi réalisé deux essais (G et H) résumés dans les tableaux suivants où les pourcentages sont exprimés en poids.

*Essai G*

| Température | + 27,5°C | | |
|---|---|---|---|
| Pression (bar abs) | 12,3 | | |
| | *mélange initial* | *phase supérieure* | *phase inférieure* |
| F 125 | 49,73 % | 80,10 % | 11,98 % |
| F115 | 2,74% | 4,23% | 0,95% |
| Perchloréthylène | 47,53 % | 15,67 % | 87,07 % |
| F115/(F115+F125) | 5,22 % | 5,02 % | 7,35 % |
| F115/F125 | 5,51 % | 5,28 % | 7,93 % |

*Essai H*

| Température | + 27,5°C | | |
|---|---|---|---|
| Pression (bar abs) | 12,3 | | |
| | *mélange initial* | *phase supérieure* | *phase inférieure* |
| F 125 | 24,16 % | 29,11 % | 10,91 % |
| F115 | 35,66% | 40,91 % | 19,79 % |
| Perchloréthylène | 40,18% | 29,98 % | 69,30 % |
| F115/(F115+F125) | 0,60 | 0,58 | 0,64 |
| F115/F125 | 1,48 | 1,41 | 1,81 |

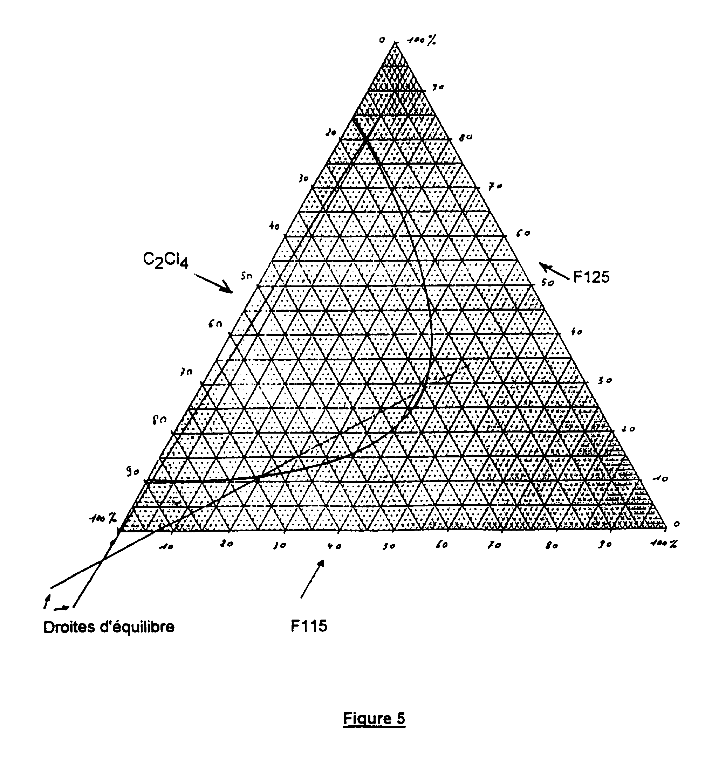

Ces essais réalisés à 27,5°C ont permis de tracer le diagramme de phase du ternaire F125/F115/perchloréthylène représenté à la Figure 5.

## EXEMPLE 3

Dans une colonne d'extraction liquide-liquide à contre-courant de 10 étages théoriques fonctionnant à 27,5°C et sous 12,3 bars absolus, on fait circuler à contre-courant :

- 436 kg/h de perchloréthylène
- 100 kg/h d'un mélange de F125 (80 % en poids) et de F115 (20 % en poids)

On obtient 498,6 kg/h d'extrait contenant la majeure partie du F115 et 37,4 kglh de raffinat pauvre en F115.
Le bilan de l'opération est résumé dans le tableau suivant.

| | Charge | | Perchloréthylène | | Extrait | | Raffinat | |
|---|---|---|---|---|---|---|---|---|
| | kg/h | %pds | kg/h | %pds | kg/h | %pds | kg/h | %pds |
| F125 | 80 | 80 | - | - | 50,1 | 10 | 29,9 | 80 |
| F115 | 20 | 20 | - | - | 18,1 | 4 | 1,9 | 5 |
| $C_2Cl_4$ | - | - | 436 | 100 | 430,4 | 86 | 5,6 | 15 |
| TOTAL | 100 | - | 436 | - | 498,6 | - | 37,4 | - |

F125/(F125+F115) dans le mélange final (Raffinat) = 94 % poids

**Revendications**

**Revendications pour les Etats contractants suivants : AT, BE, DK, GR, IE, LU, PT, SE, CH, LI**

1.  Procédé de purification d'un pentafluoroéthane (F125) contenant du chloro-pentafluoroéthane (F115) par extraction liquide-liquide ou par distillation extractive, caractérisé en ce que l'on utilise le perchloréthylène comme agent d'extraction.

2.  Procédé selon la revendication 1 dans lequel on opère sous une pression comprise entre 2 et 20 bars absolus.

**Revendications pour les Etats contractants suivants : DE, ES, FR, GB, IT, NL**

1.  Procédé de purification d'un pentafluoroéthane (F125) contenant du chloro-pentafluoroéthane (F115) par extraction liquide-liquide caractérisé en ce que l'on utilise le perchloréthylène comme agent d'extraction.

2.  Procédé selon la revendication 1 dans lequel on opère sous une pression comprise entre 2 et 20 bars absolus.

**Claims**

**Claims for the following Contracting States : AT, BE, DK, GR, IE, LU, PT, SE, CH, LI**

1.  Process for the purification of a pentafluoroethane (F125) containing chloropentafluoroethane (F115) by liquid/liquid extraction or by extractive distillation, characterized in that perchloroethylene is used as extraction agent.

2.  Process according to Claim 1, which is carried out at a pressure of between 2 and 20 bar absolute.

**Claims for the following Contracting States : DE, ES, FR, GB, IT, NL**

1.  Process for the purification of a pentafluoroethane (F125) containing chloropentafluoroethane (F115) by liquid/liquid extraction, characterized in that perchloroethylene is used as extraction agent.

2.  Process according to Claim 1, which is carried out at a pressure of between 2 and 20 bar absolute.

**Patentansprüche**

**Patentansprüche für folgende Vertragsstaaten : AT, BE, DK, GR, IE, LU, PT, SE, CH, LI**

1.  Verfahren zur Reinigung von Pentafluorethan (F125), das Chlorpentafluorethan (F115) enthält, durch Flüssig-Flüssig-Extraktion oder durch extraktive Destillation, dadurch gekennzeichnet, daß man Perchlorethylen als Extraktionsmittel verwendet.

2.  Verfahren nach Anspruch 1, dadurch gekennzeichnet, daß man bei einem Absolutdruck zwischen 2 und 20 bar arbeitet.

**Patentansprüche für folgende Vertragsstaaten : DE, ES, FR, GB, IT, NL**

1.  Verfahren zur Reinigung von Pentafluorethan (F125), das Chlorpentafluorethan (F115) enthält durch Flüssig-Flüssig-Extraktion, dadurch gekennzeichnet, daß man Perchlorethylen als Extraktionsmittel verwendet.

2. Verfahren nach Anspruch 1, dadurch gekennzeichnet, daß man bei einem Absolutdruck zwischen 2 und 20 bar arbeitet.

**Figure 1**

**Figure 2**

**SOLUBILITE DU F 125 ET F 115 DANS LE F 114**

Sélectivité: S = 2,49

F 115

F 125

Solubilité en g/l

Pression Partielle en mbar

EP 0 808 300 B1

**Figure 3**

SOLUBILITE DU F 125 ET F 115 DANS LE PERCHLORETHYLENE

Sélectivité : S = 4,0

F 115

F 125

Solubilité en g/l

Pression Partielle en mbar

**Figure 4**

EP 0 808 300 B1

Figure 5